(19) Europäisches Patentamt
European Patent Office
Office européen des brevets

(11) **EP 4 338 667 A1**

(12) **EUROPEAN PATENT APPLICATION**

(43) Date of publication:
**20.03.2024 Bulletin 2024/12**

(21) Application number: **23196835.5**

(22) Date of filing: **12.09.2023**

(51) International Patent Classification (IPC):
**A61B 5/145** (2006.01)

(52) Cooperative Patent Classification (CPC):
**A61B 5/14532;** A61B 5/4839; A61B 5/7275

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.09.2022 FR 2209157**

(71) Applicant: **Diabeloop**
**38000 Grenoble (FR)**

(72) Inventor: **LACHAL, Sylvain**
**38600 Fontaine (FR)**

(74) Representative: **Fidal Innovation**
**4-6 avenue d'Alsace**
**92400 Courbevoie (FR)**

(54) **METHOD FOR ESTIMATING AN UNANNOUNCED MEAL INGESTED**

(57)  A method for estimating a size of an unannounced meal ingested by a user, the method being implemented by a system, said system comprising:
- a storage module (301), said storage module storing a time series of glucose value of the user in a glucose storage,
- an expectation module (302), said expectation module (302) determining an expected glucose variation for a given time point,
- an estimation module (11), said estimation module (11) estimating the size of the unannounced meal ingested based on:
- a variation difference between

- a measured glucose variation between a glucose value at the given time point and a glucose value at a time point of the time series anterior to the given time point, the glucose values being acquired by a glycemia acquisition system or stored in the glucose storage;
and
- the expected glucose variation between the glucose value at the given time point and the glucose value at the time point of the time series anterior to the given time point;
- at least one user parameter relative and personalized to the user.

[Fig. 3]

EP 4 338 667 A1

**Description**

**FIELD OF THE INVENTION**

The invention relates to the field of closed-loop or open-loop systems intended for blood glucose management of a user. In particular, the invention relates to the field of estimation of a meal ingested by a user.

TECHNOLOGICAL BACKGROUND

Closed-loop systems intended for blood glucose (BG) management or control in people with type 1 diabetes (T1D), i.e. artificial pancreas (AP) systems, are still dependent on feed-forward actions such as meal announcements to achieve effective control or management. Open-loop systems intended for blood glucose management or control in people with type 1 diabetes (T1D), for example smart-pen coupled to Continuous Glucose Monitoring system, are also still dependent on feed-forward actions such as meal announcements to achieve effective control or management.

Many studies have reported that a high number of missed meal boluses occur; especially in adolescents during insulin pump therapy. For example, several studies have reported a link between glycated hemoglobin (HbA1c) levels and missed meal boluses [1-3] showing an average increase in HbAlc of 4 mmol/mol (0.3%) during a 2-week period due to missed meal boluses.

Increases in HbAlc lead to an increased risk of long-term complications such as retinopathy, nephropathy, neuropathy, heart disease, and stroke.

In healthy subjects, hypo- and hyperglycemia are counteracted by a physiological control system that includes pancreatic hormones such as insulin, glucagon, and amylin. In T1D, this control system is lacking, which is especially evident after a meal when a large postprandial increase in blood glucose is experienced. Meals are difficult to mitigate for any blood glucose management system due to the delayed onset of current rapid-acting insulin formulations and the 5-15 minutes gap inherent to CGM measurement lag between blood glucose values and interstitial glucose values measured in the interstitial space. This is a reason why those systems still need feed-forward actions from the user such as meal announcements. But, as said earlier, unannounced meals can occur and have important negative consequences.

Some systems have therefore been created to detect the occurrence of an unannounced meal ingested. Some systems are based on an optimal filtering or state observation. Others are based on anomaly detection in the blood glucose value stream combined with machine learning techniques. Fuzzy systems or moving horizon estimation systems have also been proposed.

However, the detection is only one step to reach fully autonomous blood glucose management system. Indeed, an estimation of the unannounced meal ingested detected is required to ensure a reliable blood glucose management. Current meal detection methods and systems do not estimate the size of the meal detected, or do so with a supplementary delay. The delay for estimation is around 20 minutes.

Such a delay comes from the fact that the detection is based on the integration of the glycemic rise, and on the estimation of the area under the curve instead of just the glycemic rise.

According to some methods and systems, the compensation is done through a PD controller, involving further delays.

Therefore, a better method, in particular a quicker one, to estimate the unannounced meals must be implemented.

**SUMMARY OF THE INVENTION**

The invention relates to a method for estimating a size of an unannounced meal ingested by a user, the method being implemented by a system, said system comprising:

- a storage module, said storage module storing a time series of glucose value of the user in a glucose storage,
- an expectation module, said expectation module determining an expected glucose variation for a given time point,
- an estimation module, said estimation module estimating the size of the unannounced meal ingested based on:
- a variation difference between

   a measured glucose variation between a glucose value at the given time point and a glucose value at a time point of the time series anterior to the given time point,
   the glucose values being acquired by a glycemia acquisition system or stored in the glucose storage; and
   the expected glucose variation between the glucose value at the given time point and the glucose value at the time point of the time series anterior to the given time point;

- at least one user parameter relative and personalized to the user.

The unannounced meal ingested size is calculated at the given time point $T_0$, i.e. as soon as an unannounced meal ingestion has been detected. Indeed, the data needed for the estimation of the unannounced meal are already available. This availability allows the implementation of the method already at the time point $T_0$. The method is therefore faster than the known methods which require additional data after $T_0$ for integration.

Thanks to the method, the estimation begins with the detection process or as soon as the detection has been confirmed.

Preferably, the expectation module determines the expected glucose variation at the given time point based at least on:

- an insulin sensitivity factor relative to the user, and/or
- an instantaneous consumption of insulin by the user.

Preferably, the user parameter relative and personalized to the user is based on the weight of the user.

Preferably, the system further comprises a first estimation safety module, said first estimation safety module determining

- if the measure glucose variation difference determined by the estimation module exceeds a variation difference threshold value and setting the variation difference at a value equal to the variation difference threshold value if the variation difference determined by the estimation module exceeds the variation difference threshold value.

Preferably, the system further comprises a second estimation safety module, said second estimation safety module determining if the size of the unannounced meal ingested estimated by the estimation module exceeds a meal estimation threshold value and setting the size at a value equal to the meal estimation threshold value if the size of the unannounced meal ingested estimated by the estimation module exceeds a meal estimation threshold value.

The first and second estimation safety module can be gathered into one estimation safety module determining both:

- if the measure glucose variation difference determined by the estimation module exceeds a variation difference threshold value and setting the variation difference at a value equal to the variation difference threshold value if the variation difference determined by the estimation module exceeds the variation difference threshold value; and

- if the size of the unannounced meal ingested estimated by the estimation module exceeds a meal estimation threshold value and setting the size at a value equal to the meal estimation threshold value if the size of the unannounced meal ingested estimated by the estimation module exceeds a meal estimation threshold value.

The variation threshold corresponds to a safety measure for the user. Indeed, setting a variation threshold - i.e. a maximum value for which if the variation is calculated at a superior value than this maximum value, then the variation is capped at this maximum value - allows the artificial pancreas to calculate an unannounced meal maximum insulin dose based on this maximum variation threshold. This unannounced meal maximum insulin dose cannot be too large thanks to the threshold on the variation. As the unannounced meal maximum insulin dose cannot be too large, the user does not risk being in a hypoglycemic state because of this unannounced meal maximum insulin dose.

It is a first safety measure or action.

The meal estimation threshold has a similar objective: the unannounced meal maximum insulin dose cannot be too large thanks to the threshold on the estimated size. As the unannounced meal maximum insulin dose cannot be too large the user does not risk being in a hypoglycemic state because of this unannounced meal maximum insulin dose.

It is a second safety measure or action.

Preferably, the meal estimation threshold value is calculated based on a threshold parameter determined based on the given time point.

Preferably, the system further comprises a correction module,

said correction module correcting the estimated size of the unannounced meal ingested estimated by the estimation module according to at least one correction coefficient,
the at least one correction coefficient being predetermined according to a user profile based at least on an age, a weight, a sex of the user and/or insulin needs of the user.

Preferably, the system further comprises an aggressiveness module, said aggressiveness module calculating a customized aggressiveness estimation of the size of the meal by applying an aggressiveness factor to the estimation size of the unannounced meal ingested estimated by the estimation module.

According to another aspect, the invention relates to a system for estimating a size of an unannounced meal ingested by a user comprising:

- a storage module adapted to store a time series of glucose value of the user in a glucose storage- an expectation module adapted to determine an expected glucose expected glucose variation for a given time point,
- an estimation module adapted to estimate the size of the unannounced meal ingested based on:
- a variation difference between
- a measured glucose variation between a glucose value at the given time point and a glucose value at a time point of the time series anterior to the given time point,
  the glucose values being acquired by a glycemia acquisition system or stored in the glucose storage; and
- the expected glucose variation between the glucose value at the given time point and the glucose value at the time point of the time series anterior to the given time point;
- at least one user parameter relative and personalized to the user.

In a preferred embodiment, the system further comprises a first estimation safety module adapted to

- determine if the variation difference determined by the estimation module exceeds a variation difference threshold value and to set the variation difference at a value equal to the variation difference threshold value if the variation difference determined by the estimation module exceeds the variation difference threshold value/

In a preferred embodiment, the system further comprises a second estimation safety module adapted to determine if the size of the unannounced meal ingested estimated by the estimation module exceeds a meal estimation threshold value and to set the size at a value equal to the meal estimation threshold value if the size of the unannounced meal ingested estimated by the estimation module exceeds a meal estimation threshold value,]

In a preferred embodiment, the first and second estimation safety module are gathered into one estimation safety module adapted to determine both:

- if the measure glucose variation difference determined by the estimation module exceeds a variation difference threshold value and setting the variation difference at a value equal to the variation difference threshold value if the variation difference determined by the estimation module exceeds the variation difference threshold value; and
- if the size of the unannounced meal ingested estimated by the estimation module exceeds a meal estimation threshold value and setting the size at a value equal to the meal estimation threshold value if the size of the unannounced meal ingested estimated by the estimation module exceeds a meal estimation threshold value.

In a preferred embodiment, the meal estimation threshold value being calculated based on a threshold parameter determined based on the given time point.

In a preferred embodiment, the system further comprises a correction module adapted to correct the estimated size of the unannounced meal ingested estimated by the estimation module according to at least one correction coefficient, the at least one correction coefficient being predetermined according to a user profile based at least on an age, a weight, a sex of the user and/or insulin needs of the user.

In a preferred embodiment, the system further comprises an aggressiveness module adapted to calculate a customized aggressiveness estimation of the size of the meal by applying an aggressiveness factor to the estimation size of the unannounced meal ingested estimated by the estimation module.

According to another aspect, the invention relates to a computer program for estimating a size of an unannounced meal ingested by a user wherein the computer program is adapted, when run on a processor, to cause the processor to implement the method of the invention.

The computer program is adapted, when run on a processor, to cause the processor to implement each step of the method of the invention.

## BRIEF DESCRIPTION OF THE DRAWINGS

Embodiments of the invention will be described below, in relation to the following drawings:

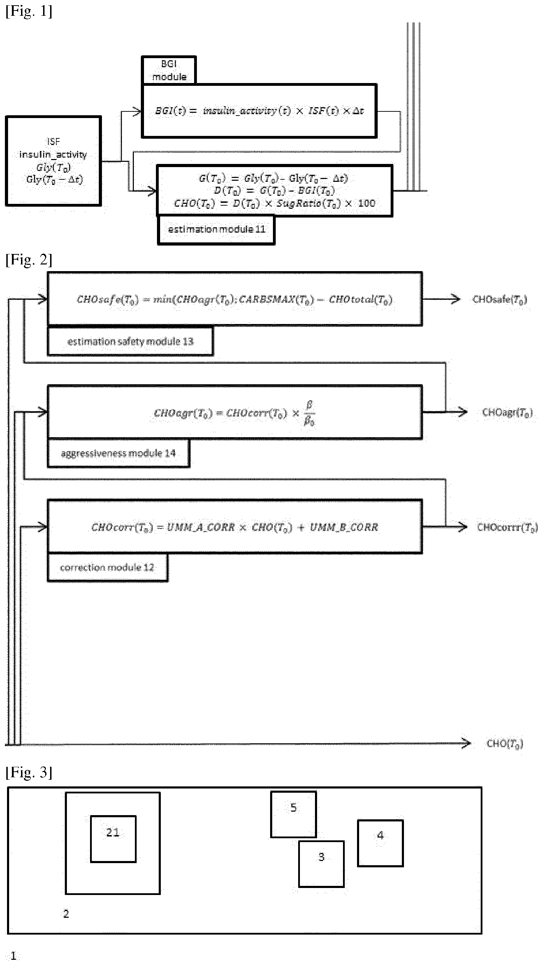

[Fig.1] and 2 illustrate an algorithmic possible organization (and some variations) of the method of estimation.
[Fig.1] is the first part of the algorithmic organization and [Fig.2] is the second part.

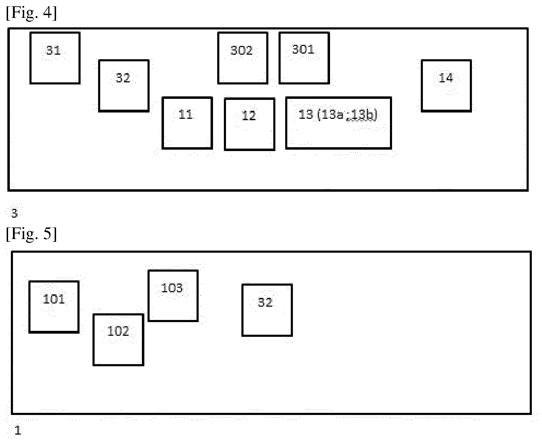

[Fig.3] and [Fig.4] illustrate a possible embodiment of an artificial pancreas 1 implementing a method of estimation. [Fig.5] illustrates a possible embodiment of an artificial pancreas 1 comprising the modules required for estimating an amount of insulin needed to compensate for an unannounced meal ingested by a user.

On the drawings, the same reference signs show the same or similar objects.

DEFINITIONS

Unit of insulin

In the present detailed description and according to the WHO Expert Committee on Biological Standardization one international unit of insulin (1 U) is defined as the "biological equivalent" of 34.7 micrograms ($\mu$g) pure crystalline insulin. This unit is the relevant unit for discussing a quantity of insulin to be infused to a user, and cannot be converted to the International System of Units, because the conversion would depend on which insulin is being used. For the sake of the disclosure of the present invention, it is important that quantities of insulin be expressed in a system meaningful for the invention, the readers and the scientific community.

Closed-loop and open-loop system

A closed loop control system is a set of mechanical or electronic devices that automatically regulate a process variable to a desired state or set point without human interaction. Closed loop control systems contrast with open loop control systems, which require manual input.

Insulin Sensitivity Factor (ISF)

An insulin sensitivity factor (ISF) or correction factor describes how much one unit of rapid or regular insulin will lower blood glucose concentration. For example:

ISF of 1 means: 1 unit of insulin lowers glucose by 1 mmol/L
ISF of 2 means: 1 unit of insulin lowers glucose by 2 mmol/L
ISF of 3 means: 1 unit of insulin lowers glucose by 3 mmol/L

Insulin-to-Carb Ratio (ICR)

Insulin-to-Carb Ratio (ICR) equals the number of grams of carbohydrates that 1 unit of rapid-acting insulin will cover.

DETAILED DESCRIPTION

General overview

An artificial pancreas 1 is a system that automatically regulates the insulin intake of a diabetic user based on its blood glucose history, meal history, and insulin history.

An artificial pancreas 1 is often a model-based artificial pancreas with various modules. Those modules can be computerized. From a most general point of view, four modules can be distinguished in the artificial pancreas 1:

- a data acquisition system 2,
- a data processing system 3 comprising or in communication with a blood glucose prediction module 31 and an insulin dose (and possibly also glucagon dose) calculation module 32. Those two modules can be gathered in a data processing system 3 or only communicate with each other,
- an active system 4 adapted to deliver an insulin or glucagon dose
- a controller system 5.

The data processing system 3 comprises a clock adapted to determine time and any duration between time points.

The data acquisition system 2 is responsible for the implementation of the different sensors worn by the user or in communication with the artificial pancreas 1. It can manage a continuous glucose monitoring (CGM) system or be in communication with it. The objective of the data processing system 3 is to process the various data collected from the data acquisition system 2 and to output an insulin dose (or glucagon dose) to the active system 4.

The objective of the blood glucose prediction module 31 is to predict future blood glucose based on the current blood glucose, and the physiological parameters or variables able to influence the trend of the blood glucose.

The blood glucose prediction module 31 is adapted to determine a prediction of a temporal value. This prediction is a prediction data value. Here the temporal value is the blood glucose.

To determine the prediction data value, the blood glucose prediction module 31 can be adapted to implement a predictive model. The blood glucose prediction module 31 can be based on a physiological predictive model or on a deep-learning or machine learning predictive model.

For example, variables of the blood glucose prediction module 31 can be a current value of active insulin in the user's body, the carbohydrates ingested (CHO) or the meal history, and physiological parameters used by the blood glucose prediction module 31 can be the insulin sensitivity factor (ISF) or the carbohydrate-to-insulin ratio (CIR). The blood glucose prediction module 31 is responsible for predicting a hypoglycemic or hyperglycemic state of the user.

This predictive model is adapted to determine, calculate or compute a prediction P, i.e. a predicted value P. This prediction is the output of the model from an algorithmic point of view. It can be named the predictive output of the model. This prediction is made about a physical characteristic of a time-based system, varying over time, i.e. a time-based parameter (or characteristic) representative of the time-based system. Here the time-based variable or parameter is the blood glucose.

In addition, this prediction is made for a predetermined future time point.

By convention, starting from an initial time point $T_0$, a prediction P $\Delta t$ time ahead is a temporal prediction $P_x$ made at the initial time point $T_0$ for a future time point $T_t = T_0 + \Delta t$ where $\Delta t$ is a duration.

Here, the blood glucose prediction module 31 calculates, computes or outputs a blood glucose prediction PGly at a constant predetermined time interval ahead, for example one, two, five or ten minutes. The prediction PGly can be for any duration $\Delta X$ ahead. For example, it can be five minutes, ten minutes, fifteen minutes, thirty minutes ahead. Multiple predictions made at a same initial time point are also possible.

The objective of the insulin dose (and possibly also glucagon) calculation module 32 is to determine if an insulin (or glucagon) dose is required based on the current blood glucose and the predicted blood glucose calculated by the blood glucose prediction module 31 to maintain or reach a target value for the blood glucose or a target range for the blood glucose and, if so, to calculate a proper insulin (or glucagon) dose to maintain or reach the target value for the blood glucose or the target range for the blood glucose.

The active system 4 is responsible for delivering the insulin (or glucagon) based on the insulin (or glucagon) dose calculated by the insulin dose calculation module 32. The active system 4 comprises an infusion system. For example, the insulin dose calculation module 32 is remote from the active system 4, and is adapted to communicate with it through any suitable means, such as wireless (radiofrequency, such as Bluetooth) or wiredly.

The controller system 5 is responsible for coordinating and controlling the different modules of the artificial pancreas 1.

In particular, the present invention relates to the meal history and especially the unannounced meals ingested.

As described above, the meal history is taken into account by the blood glucose prediction module 31. An instant meal ingested can also be taken into account, but as said earlier, this requires an input from the user. If the user does not input that a meal was ingested and what amount of carbohydrates ("CHO") has been ingested, the prediction made by the blood glucose prediction module 31 is likely to be wrong.

The lack of detection and estimation of an unannounced meal ingested leads to a rise of the blood glucose only taken into consideration when detected by the CGM system. Then, the insulin dose calculation module 32 calculates an insulin dose based on the rise of the blood glucose due to the unannounced meal ingested.

This process can be too slow to prevent a hyperglycemic state for the user.

It is therefore desirable to be able to improve the performance of the artificial pancreas 1, and more particularly to be able to improve the accuracy and the reactivity of the prediction model in order to better estimate insulin requirements and reduce the risk of hyperglycemia.

**Solution - estimation of the unannounced meal and compensation of it**

**Method of estimation**

Components of the general system implementing the method of estimation

The components required by the general system, i.e. the artificial pancreas 1, implementing the method of estimation are the ones previously presented in the general overview:

- a data acquisition system 2;
- a data processing system 3 adapted to implement any kind of modules as described later;

- a controller system 5.

## Modules for the method of estimation

Various modules can be implemented by the data processing system 3 of the general system, i.e. the artificial pancreas 1:

- a blood glucose or glycemia acquisition module 21 adapted to receive a glycemia signal Gly from a glycemia acquisition system and to store it in a storage module 301,
- an expectation module 302 adapted to determine an expected glucose expected glucose variation for a given point by determining an expected glucose expected glucose and comparing it to the current blood glucose,
- an estimation module (CHO module) 11 a correction module (CHOcorr module) 12, an aggressiveness module (CHOagr module) 14, an estimation safety module (CHO safe) 13, all modules being adapted to operate any mathematical operation on the values from the signals received from other modules of the artificial pancreas 1 or on values of various parameters stored in the memory of the data processing system 3. In particular, the previous modules are adapted to realize addition, subtraction, division and multiplication.

Those modules are computerized.

The blood glucose or glycemia acquisition module 21 is adapted to receive a glycemia signal Gly from a glycemia acquisition system at a predetermined time interval. For example, this time interval can be one, two, five minutes or any duration between one and ten minutes.

## Method of estimation - General proposition

It is assumed that a meal has already been detected.

The object of the method is to estimate the size of the meal which is either unannounced or announced with no declaration of an amount of carbohydrates. The suggestion of the method is to convert a blood glucose deviation into a meal, or carbohydrates (CHO) estimation.

Preferably, the conversion occurs immediately after the deviation has been detected leading to an instantaneous estimation.

The data processing system 3 also has in its memory a set of variables relating to the user or various temporal data field: an ISF parameter which can be re-evaluated from time to time, the history of delivered insulin to the user, the insulin on board (IOB) of the user determined with the history of delivered insulin.

Those variables can vary over time according to updates of the set of parameters. However, at the time point when the method is implemented, those variables are fixed and determined.

The proposition of the invention is to, at a given time point of the time series of blood glucose, compare the latest blood glucose measurements with the past blood glucose measurements and detect a deviation between the latest blood glucose measurements and the expected glucose expected glucose expected glucose measurements. Then, the proposition is to use this deviation to estimate the size of the unannounced meal which has caused the deviation.

It is assumed that the storage module has stored a time series of blood glucose measurements received over time from the blood glucose data acquisition module 21.

Based on those blood glucose measurements, possibly a declared ingested meal, and the set of various parameters relating to the user, the expectation module 302 determines an expected glucose expected glucose expected glucose variation of the user.

This expected glucose expected glucose expected glucose variation can be determined by various approaches.

For example, the expectation module 302 determines the expected glucose expected glucose expected glucose variation at the given time point based at least on:

- an insulin sensitivity factor relative to the user, and
- an instantaneous consumption of insulin by the user.

In another example, the glucose variation can be determined by calculating an expected glucose expected glucose expected glucose for a future time point and compute the expected difference between the expected glucose expected glucose expected glucose and the first posterior blood glucose measurement. Then, this difference can be compared with the difference between the current blood glucose and the first posterior blood glucose measurement (i.e. two consecutive blood glucose measurements).

This expected glucose expected glucose expected glucose can be determined at any future time point. For example, the expected glucose expected glucose expected glucose can be determined for a future time point 5, 10, 15, 20, 25, 30 minutes ahead, 5 to 30 minutes ahead. The future time point is the given time point when the method is implemented.

Based on this expected glucose expected glucose expected glucose for the future time point, i.e. the given time point, the expectation module 302 compares the expected glucose expected glucose expected glucose to the current blood glucose at the future time point, i.e. at the given time point and determines the expected variation.

By extension, the expected variation can be determined for a future time point 5, 10, 15, 20, 25, 30 minutes ahead, 5 to 30 minutes ahead.

Based on this expected variation, the proposition is to detect a deviation between the current glucose variation and the expected glucose expected glucose expected glucose variation at the given time point.

Thanks to this feature, the method allows an estimation of the unannounced meal as soon as it is detected.

The deviation can be determined by a dedicated deviation module, the expectation module 302 or the estimation module 11.

In other words, the deviation is a variation difference between:

- a measured glucose variation between a blood glucose value at the given time point and a blood glucose value at a time point of the time series preceding the given time point, and
- the expected variation of the blood glucose for the given time point.

Then, based on this variation, at least one stored blood glucose value for the time point preceding the given time point and at least one user parameter relative and personalized to the user, the estimation module 11 estimates the size of the unannounced meal ingested.

Preferably, the user parameter relative and personalized to the user is based on the weight of the user as it will be described more precisely in a detailed embodiment.

Based on this expectation, the blood glucose management system determines an insulin amount to be delivered to the user. An overestimation can therefore lead to an insulin amount too large which could lead to a hypoglycemic state for the user. To avoid such dangerous state for the user, the method proposes to implement a first estimation safety module 13a, a second estimation safety module 13b, or an estimation safety module 13 gathering the first and second estimation safety modules 13a and 13b.

The estimation safety modules 13a and 13b have for objective to avoid a too big estimation of the size of the unannounced meal. To do so, the estimation safety modules 13a and 13b monitor two thresholds:

- the first estimation safety module 13a monitors a variation threshold relative to the variation difference,
- the second estimation safety module 13b monitors a meal estimation threshold relative to estimated size of the unannounced meal.

The estimation safety modules 13a and 13b monitor the determined variation difference and the estimated size and sets the determined values of the variation difference and the estimated size value at the threshold values.

Those threshold values can be determined for the user.

Thus, the estimation safety module 13a determines:

- if the variation difference determined by the estimation module 11 exceeds a variation threshold and set the variation difference at this threshold in this case.

Likewise, the estimation safety module 13b determines :

- if the size of the unannounced meal ingested estimated by the estimation module 11 exceeds a meal estimation threshold and sets the size at this threshold in this case.

The estimation safety modules 13a and 13b can be gathered into one estimation safety module 13 which implements the same monitoring of the estimation safety modules 13a and 13b. It can also implement only the monitoring of the estimation safety module 13a or only the monitoring of the estimation safety module 13b.

The meal estimation threshold can be calculated based on a threshold parameter determined based on the given time point.

In addition to those safety measures, the method can also implement a correction module 12 and an aggressiveness module 14 to personalize the estimation to the user.

The correction module 12 is configured to correct the estimated size of the unannounced meal ingested estimated by the estimation module 11 according to at least one correction coefficient where the at least one correction coefficient is predetermined according to a user profile based at least on an age, a weight, a sex of the user and/or insulin needs of the user.

This correction module 12 allows a personalization of the estimation to the user thanks to the at least one correction coefficient.

In a preferred embodiment, two correction coefficients are used by the correction module 12 to correct the estimated size with a linear correction.

The aggressiveness module 14 is configured to calculate a customized aggressiveness estimation of the size of the meal by applying an aggressiveness factor to the estimation size of the unannounced meal ingested estimated by the estimation module 11.

The aggressiveness factor can also be personalized to and by the user.

Those two personalization steps implemented by the correction module 12 and the aggressiveness module 14 can be combined in all possible ways: first, the correction measure then the aggressiveness measure or the contrary.

Detailed embodiment

In this embodiment, the estimation module 11 implements a blood glucose impact module to determine the expected glucose expected glucose expected glucose variation of the user.

The blood glucose impact module calculates the blood glucose impact (BGI). The blood glucose impact corresponds to the expected glucose expected glucose expected glucose at a given time point or moment. The BGI can be seen as the instantaneous expected glucose expected glucose expected glucose variation.

For example, the BGI is calculated according to the following formula:

$$BGI(t) = insulin\_activity(t) \times ISF(t) \times \Delta t$$

where:

insulin_activity(t) is determined with the insulin on board at a time point t,
ISF(t) is the user's Insulin Sensitivity Factor [mg/dL/U] at a time point t, and
$\Delta t$ is a time interval between the given time points.

The insulin activity can be set as the instantaneous insulin consumption or disappearance.

If the insulin activity is measured as negative, its value is set at zero.

One model describes the gradual disappearance of insulin after a bolus injection, generally in the form of a bell (slow disappearance shortly after injection, then peak insulin absorption, then slow consumption long after injection). The variable IOB(t) is the total amount of insulin available, and insulin activity is the opposite of its derivative, the instantaneous disappearance of insulin. This insulin activity is derived from the one model, and fed by the timings and quantities of insulin injected. When insulin is injected, the variable IOB(t) increases sharply, so the opposite of its derivative is artificially negative for an instant and so its value is set at zero.

This setting at zero for a negative insulin activity is also a security measure or step, an insulin activity inferior to 0 has no physical sense, so it is fixed at 0.

Thus:

$$BGI(T_0) = insulin\_activity(T_0) \times ISF(T_0) \times (T_0 - (T_0 - \Delta t))$$

Then, the estimation module 11 of the data processing system 3 implements the following formulas at the time point:

- a difference $G(T_0)$ between a glycemia value at $T_0$ from the glycemia signal and a glycemia value at the time point preceding $T_0$ in the time-series $Gly(T_0 - \Delta t)$:

$$G(T_0) = Gly(T_0) - Gly(T_0 - \Delta t)$$

- a difference $D(T_0)$ between the glycemia value $G(T_0)$ and the BGI value at $T_0$ $BGI(T_0)$:

$$D(T_0) = G(T_0) - BGI(T_0)$$

- an estimated value of the unannounced meal ingested CHO by calculating the following formula:

$$CHO(T_0) = D(T_0) \times SugRatio(T_0) \times 100$$

where *SugRatio(t)* is a predetermined Sugaring Ratio [g/g/L] based on the weight of the user.

The sugaring ratio is a function of the weight known from diabetes research for a portion of carbohydrates of 20 g CHO.

In this embodiment, a sugaring ratio formula allows to accurately determine a sugaring ratio for a person with a weight between 20 and 220kg and then use this sugaring ratio to determine the value of the unannounced meal ingested CHO.

The bound values for the sugaring ratio are 5 and 90 for example.

The ingestion of the meal being already detected as assumed, $G(T_0)$ is likely to be positive.

However, as the detected meal was not planned, $G(T_0)$ is also likely to be greater than $BGI(T_0)$ and therefore $D(T_0)$ to be positive.

From a positive $D(T_0)$, the unannounced meal ingested $CHO(T_0)$ is estimated by the above-mentioned formula.

Then, as will be described later, the estimated size is used to manage an upcoming rise of glucose. It can be used to control the delivery of insulin and/or glucagon in order to manage the blood glucose of the user.

Additional modules

Some improvements will now be described. Those improvements can be combined together and are implemented by the following additional modules: the estimation safety module 13 (13a; 13b) the correction module 12 and the aggressiveness module 14.

In a one embodiment, the estimation safety module 13a implements a hypoglycemia safety measure.

Indeed, this hypoglycemia safety measure is used to prevent a too large response from the insulin dose calculation module 32 after the estimation of the unannounced meal ingested. The insulin dose calculated with a large amount of CHO estimated could lead to a hypoglycemia if the CHO estimation was not accurate.

To implement this hypoglycemia safety measure, a first safety step compares the difference $G(T_0)$ to a predetermined safety threshold. This predetermined safety threshold can be called *UMM_DELTA_G_MAX_SLOPE*. Thus, the first safety step determines whether

a)

$$G(T_0) > UMM\_DELTA\_G_{MAX_{SLOPE}}$$

Or
b)

$$G(T_0) < UMM\_DELTA\_G_{MAX_{SLOPE}}$$

If condition a) is verified, then, the first safety step sets $G(T_0)$ to *UMM_DELTA_G_MAX_SLOPE.*

In other words, the difference $G(T_0)$ is bounded at an upper limit which avoids a too large insulin response by the insulin dose calculation module 32.

The predetermined safety threshold UMM_DELTA_G_MAX_SLOPE is preferably comprised between two and six mg/dL/min and more preferably the predetermined safety threshold is set at two, three four, five or six mg/dL/min.

The estimation safety module 13b implements a second safety step: a maximum size measure. According to this measure, the estimated size is capped according to a maximum size CARBSMAX. Even if the deviation leads to the determination of the estimated size greater than the maximum size CARBSMAX, the second safety step sets the value of the estimated size at CARBSMAX.

For example, during the second safety step, the estimation safety module 13 calculates a hypoglycemia safety estimated ingested meal CHOsafe according to the following formula:

$$CHOsafe(T_0) = min(CHO(T_0), \ CARBSMAX - CHOtotal)$$

where

*CHOtotal* is the sum of the estimation CHO, CHOcorr or CHOagr since the last meal detection, and CARBSMAX can take two predetermined values
*UMM_MAX_ESTIM_CHO_PRANDIAL* and *UMM_MAX_ESTIM_CHO_OUT_PRANDIAL* depending on the time of day:

- *CARBSMAX = UMM _MAX_ESTIM _CH0 _PRAN DI AL* for meal time,
- *CARBSMAX = UMM_MAX_ESTIM_CHO_OUT _PRANDIAL* on other time periods.

As an example, the meal time periods correspond to time periods [11:00; 14:00] and [18:00; 22:00].

As an example, the predetermined value UMM_MAX_ESTIM_CHO_PRANDIAL is comprised between 30 and 70 and

As an example, the predetermined value UMM_MAX_ESTIM_CHO_OUT_PRANDIAL is comprised between 10 and 50.

The estimation safety modules 13a and 13b can be gathered into one estimation safety module 13 or two distinct modules. If the estimation safety modules 13a and 13b are distinct, it is possible to implement only one of the two.

The correction module 12 aims at personalizing the estimation to the user.

To do so, the correction module 12 corrects the estimated size according to at least one correction factor.

For example, the correction module 12 calculates an improved estimation CHOcorr by applying a linear meal estimation correction model to the ingested meal CHO defined by the following formula:

$$CHOcorr(T_0) = UMM\_A\_CORR \times CHO(T_0) \ + \ UMM\_B\_CORR$$

where
the coefficients *UMM_A_CORR* and *UMM_B_CORR* are predetermined according to a user profile based at least on an age, a weight, and/or a sex of the user.

For example, for teen male users of average weight, *UMM_A_CORR* = 2.5 and *UMM_B_CORR* = 1.

UMM_B_CORR can be equal to zero. In this case, the correction module 12 only uses one correction factor.

In other words, this allows a simple personalization to the user based on a personalization of the parameters according to a population classification.

In an embodiment, the estimation of the ingested meal $CHO(T_0)$ can be parametrized or customized to user (and possibly by the user) with an aggressiveness factor relative to the user denoted as $\beta$. This aggressiveness factor relative to the user is to be compared to a standard aggressiveness factor $\beta_0$.

For example, $\beta$ can be customized by the user in the range [0.1; 2] and preferably in the range [0.3 ; 1.3].

For example, $\beta_0$ is a standard aggressiveness factor whose value is in the range [0.1; 2] and preferably 0.7.

The aggressiveness module 14 calculates a customized aggressiveness estimation CHOagr by applying an aggressiveness factor to the estimation $CHO(T_0)$ according to the following formula:

$$CHOagr_{CHO}(T_0) = CHO(T_0) \ \times \ \frac{\beta}{\beta_0}$$

As said before, the embodiments can be combined together. In other words, the aggressiveness module 14 can calculate a customized aggressiveness estimation CHOagr by applying an aggressiveness factor to the improved estimation $CHOcorr(T_0)$ according to the following formula:

$$CHOagr_{CHO_{corr}}(T_0) = CHOcorr(T_0) \times \frac{\beta}{\beta_0}$$

Those different embodiments are illustrated by the different variations in [Fig.2] in the relationships between the different modules.

As said earlier, the embodiments can be combined. Therefore, the estimation safety module 13 can implement the second safety step on the improved estimation CHOcorr applied to the estimation CHO, on the customized aggressiveness estimation CHOagr applied to the estimation CHO and even on the customized aggressiveness estimation CHOagr applied to the improved estimation CHOcorr. Those combinations correspond to the following formulas:

$$CHOsafe(T_0) = min(CHOcorr(T_0), CARBSMAX - CHOtotal)$$

$$CHOsafe(T_0) = min(CHOagr_{CHO}(T_0), CARBSMAX - CHOtotal)$$

$$CHOsafe(T_0) = min(CHOagr_{CHO_{corr}}(T_0), CARBSMAX - CHOtotal)$$

Those different embodiments are also illustrated by the different variations in [Fig.2] in the relations between the different modules.

Second embodiment

This embodiment only differs in the way the expected glucose expected glucose variation at the given time point $T_0$ is determined.

In this previous embodiment, the blood glucose impact corresponds to the instantaneous expected glucose expected glucose variation at a given time point or moment.

In this other embodiment, the expected glucose expected glucose is determined by the blood glucose prediction module 31.

As described earlier, the blood glucose prediction module 31 can compute a prediction PGly $\Delta X$ time ahead at a constant time interval. The reliability of this prediction can be tested during the duration $\Delta X$. It is assumed that the reliability of the prediction has been assessed to be used by the data processing system 3.

In this other embodiment, the blood glucose prediction module 31 outputs each five minutes, a prediction PGly thirty minutes ahead. Thus, at a given time point $T_0$ the blood glucose prediction module 31 has already computed a prediction PGly($T_0$) made for the time point $T_0$.

This prediction PGly($T_0$) could have been made at any previous preceding time point as long as its reliability has been assessed.

Meanwhile, as described in the first embodiment, the blood glucose or glycemia acquisition module 21 adapted to receive a glycemia signal Gly from a glycemia acquisition system receives the signal from the data acquisition system 2.

Thus, for a same time point, the data processing system 3 has in its memory the prediction PGly made for this time point and the current blood glucose value Gly.

According to the second embodiment, the difference D($T_0$) is calculated with the following formula:

$$D(T_0) = G(T_0) - PGly(T_0)$$

Following this difference between the first embodiment and the second embodiment, CHO($T_0$) is calculated by the estimation module 11 according to the following formulas

$$CHO(T_0) = D(T_0) \times SugRatio(T_0) \times 100$$

$$CHO(T_0) = \left(G(T_0) - PGly(T_0)\right) \times SugRatio(T_0) \times 100$$

Advantages of the method of estimation

The method of estimation solves the above-mentioned drawbacks of the state of art.

As a reminder, the concentration of blood glucose over time in a subject is considered a time-based system with a temporal inertia of approximately 30 minutes. In other words, there is a 30-minutes delay between an insulin injection and the corresponding effect on the blood glucose.

In particular, the method allows an immediate estimation of the unannounced meal since the estimation starts as soon as the detection of an unannounced meal is confirmed.

This immediate estimation is allowed by taking into consideration the difference D which is based on the current blood glucose value $Gly(T_0)$ and past values.

Since the temporal inertia is approximately 30 minutes, tolerating a delay for the algorithmic management of the unannounced meal leads to inevitable blood glucose rises which can have bad consequences for the health of the user.

In addition, by immediately estimating the unannounced meal ingested, the method allows an improvement of the prediction of future blood glucose and therefore a better calculation of the insulin dose required to reach or maintain blood glucose in the target range or at the target value.

In other words, the method allows a virtuous algorithmic circle.

As detailed before, the method allows to manage the upcoming rise since the first period of time, here for example 5 minutes, and thus allows a smooth management of the blood glucose.

**Compensation**

The compensation represents the management of the blood glucose by the artificial pancreas 1.

The compensation is implemented by a system including an active system or a distant server i.e. a server in communication with the system of a user.

Components

The method and system according to the invention can be used to improve the management of the blood glucose of people with type 1 diabetes (T1D).

The components required by the general system, i.e. the artificial pancreas 1, implementing the compensation are the ones previously presented in the general overview:

- a data acquisition system 2;
- a data processing system 3 adapted to implement any kind of modules as described later;
- a controller system 5.

However, the artificial pancreas 1 requires an additional component compared with the components used for the method of estimation: an active system 4, which comprises an infusion system.

It is also possible that the artificial pancreas 1 has an interface system which allows the user to see some visual outputs of the data processing system 3 or to input some announcements to the data processing system 3.

For example, the data processing system 3 is remote from the active system 4, and is adapted to communicate with it through any suitable means, such as wireless (radiofrequency, such as Bluetooth) or wired.

The method for estimation of the size of an unannounced meal can be implemented periodically by the blood glucose management system. This estimation is preferably implemented each five minutes.

Then, the estimated size, whether or not it is improved by any additional modules developed above, is then used to manage the blood glucose of the user.

Based on the estimated size of the unannounced meal, the blood glucose management system determines or estimates an insulin bolus to counter the expected rise of glucose due to the unannounced meal.

Modules of the system

The compensation can be implemented by a system comprising various modules implemented by the data processing system 3.The system can be a terminal device or a distant server in communication with a terminal device.

Those modules are the followings:

- a detection module 101 adapted to or configured to detect an unannounced meal at an initial time-point,
- an estimation module 102 adapted to or configured to estimate the size of the unannounced meal detected by the detection module 101,
- a compensation module 103 adapted to or configured to calculate an amount of insulin to compensate for the detected and estimated unannounced meal.

The detection module 101 can detect an unannounced meal according to any methods.

In one embodiment, the detection is based on a deviation between an expected glucose expected glucose variation and the current blood glucose variation. The variation difference between a measured glucose variation between a glucose value at the given time point and a glucose value at a time point of the time series anterior to the given time point used by the estimation module 102 implemented for the method of estimation can be used by the detection module 101 implemented for the compensation.

The estimation module 102 can be one of the following previously presented modules: an estimation module (CHO module) 11, a correction module (CHOcorr module) 12, an aggressiveness module (CHOagr module) 14, an estimation safety module (CHO safe) 13.

As described above, all of these modules are adapted to estimate the size of the unannounced meal based on:

- a variation difference between:
- a measured glucose variation between a blood glucose value at the given time point and a blood glucose value at a time point of the time series preceding the given time point, and
- the expected variation of the blood glucose for the given time point.
  and
- at least one user parameter relative and personalized to the user.

The compensation module 103 is adapted to or configured to calculate or estimate an amount of insulin based on the estimated size of the unannounced meal.

Any method can be implemented to estimate or calculate the amount of insulin required to lower the blood glucose after its rise due to the unannounced meal or, better, to anticipate the blood glucose rise due to the unannounced meal.

In particular embodiments, the system can also comprise a meal size safety module 23 adapted to determine if the estimated size of the unannounced meal exceeds a meal estimation threshold value and setting the estimated size at a value equal to the meal estimation threshold value if the estimated size of the unannounced meal exceeds the meal estimation threshold value. This meal size safety module 23 can be the estimation safety module 13.

In another particular embodiment, the system further comprises a condition module 15 adapted to supervise the compensation and especially the calculation step. The condition module 15 is adapted to verify whether at least one condition is met, wherein one of the at least one condition is: a variable IOB(t) representative of the time variation of the user's quantity of insulin on board is lower than a predetermined value and/or a total amount of insulin calculated during a predetermined past time period is lower than a predetermined value.

If this at least one condition is met, then the calculation step is implemented by the compensation module 103.

In a particular embodiment, the amount of insulin calculated by the compensation module 103 can comprise one or more of the followings components:

- a residual component based on at least one previous estimated size of a previous unannounced meal and a predetermined ingestion profile relative to and personalized to the user.
- a meal component calculated according to a predetermined meal ratio and the estimated size of the unannounced meal;
- a net IOB component calculated according to a net insulin-on-board of the user.

The meal component is calculated according to a predetermined meal ratio and the estimated size of the unannounced meal. For example, the following formula can be used:

$$Meal\ component\ = EstimatedSizeMeal \times mealRatio$$

The meal component aims at compensating only the unannounced meal.

The residual component aims at compensating a residual part of a previous unannounced meal which would not have been totally compensated by a previous amount of insulin calculated by the compensation module 103.

First, the compensation module 103 has to evaluate how much of the last estimated unannounced meal ingested has been diffused in the blood of the user since the last estimation. To do so, a user specific diffusion curve linking an amount (CHO) of an ingested meal and the corresponding diffusion time into the blood is used. According to the diffusion curve, the totality of an ingested meal is diffused into the blood after a user specific duration D.

The diffusion curve can be personalized to the user based on calibration measurements such as the user specific duration D but it can also be a user-type diffusion curve to be chosen between a predetermined number of user-types or user categories.

Based on the diffusion curve, the compensation module 103 might evaluate that a residual part of the last estimated unannounced meal has not been completely compensated by the last amount of insulin calculated. As an example, the following formula can be used:

$$Residual\ Meal\ (t) = InitialEstimatedMealSize\ (t - \Delta t) - DiffusedSize\ (\Delta t)$$

From an initial moment of the estimation of the unannounced meal size, all the residual meal amounts for the user specific duration D are calculated based on the diffusion curve with a different $\Delta t$ for each residual meal. Then, based on all the residual amounts estimated, the compensation module 103 estimates the residual component to compensate for the sum of the residual amounts estimated.

The initial moment of the estimation of the unannounced meal size is sliding with a $\Delta t$ time-step.

The net IOB component calculated by the compensation module 103 reflects the past injected insulin still active in the body which needs to be accounted for to avoid possible insulin overdosing. It is relative to the baseline IOB insulin corresponding to the user reference insulin basal.

A positive value for the net IOB means active insulin above baseline while a negative value means active insulin below baseline. This value of the net IOB component is subtracted from the amount of insulin calculated by the compensation module 103 only in case of positive or null value to allow the compensation module 103 to reduce the amount of insulin calculated.

The net IOB component is therefore a null or negative component of the amount of insulin calculated by the compensation module 103.

In addition, in the previous particular embodiment, the amount of insulin calculated by the compensation module 103 can also comprise one other component defined as a blood glucose component.

The blood glucose component aims at maintaining the blood glucose of the user in a user target blood glucose range or at a user blood glucose target according to the current blood glucose measured (or received) prior to the detection of the unannounced meal ingested.

The compensation module evaluates if the current blood glucose prior to the detection of the unannounced meal is below a predetermined hypoglycemia limit (for example comprised between 70 and 85 mg/dL) or over a user predetermined blood glucose target.

In such occurrence, the compensation module 103 estimates the blood glucose component amount of insulin based on the current blood glucose prior to the detection of the unannounced meal, the user predetermined blood glucose target (or the boundaries of the blood glucose) and a determined compensation ratio.

For example, the blood glucose component of the amount of insulin can be estimated by the following formula:

$$BG\ Component = (BG\ prior) \times compensation\ ratio$$

If the current blood glucose prior to the detection of the unannounced meal was below the predetermined hypoglycemia limit, then the previous formula leads to a negative value for the blood glucose component which means that the amount of insulin will be reduced according to the negative value of the blood glucose component.

If the current blood glucose prior to the detection of the unannounced meal was over a user predetermined blood glucose target, then the previous formula leads to a positive value for the blood glucose component which means that the amount of insulin will be increased to reach the blood glucose target and compensate the unannounced meal (with the meal component).

If the current blood glucose prior to the detection of the unannounced meal is not comprised between a predetermined hypoglycemia limit (for example comprised between 70 and 85 mg/dL) or and over a user predetermined blood glucose target then, the blood glucose component is equal to zero.

Steps of the compensation

From an algorithmic point of view, the compensation has for input an amount of ingested carbohydrates (CHO) and for output an amount of insulin to compensate for the ingested carbohydrate.

The method starts as soon as the unannounced meal ingested has been estimated by the estimation module 102 which can implement any embodiment of the method of estimation.

As described earlier, the method to estimate the size of an unannounced meal can be implemented periodically by the blood glucose management system. This estimation is preferably implemented each five minutes.

Then, the compensation module 103 implements a calculation step consisting of calculating an amount of insulin at least based on the estimated size of the unannounced meal.

Any method can be used to estimate or calculate the amount of insulin.

However, the compensation can be supervised by the estimation safety module 23 and/or the condition module 15.

The estimation safety module 23 is adapted to determine if the estimated size of the unannounced meal exceeds a meal estimation threshold value and setting the estimated size at a value equal to the meal estimation threshold value if the estimated size of the unannounced meal exceeds the meal estimation threshold value.

It can be seen as an entry supervisor since the estimation safety module 23 supervises the algorithmic entry of the compensation.

Thus, by controlling the maximum entry value of the compensation module 103, the amount of insulin estimated by the compensation module 103 is also controlled. This is a first safety measure.

On the other hand, the condition module 15 can be seen as an output supervisor since it is adapted to verify if at least one condition is met before implementing the compensation and especially the calculation step and/or before recommending to the user the estimation provided by the compensation module 103.

This condition can also be seen as a safety requirement. To ensure safety of the user, it can be relevant to monitor the insulin-on-board of the user (IOB). The at least one condition can also be related to the total amount of insulin calculated during a past predetermined duration, for example the last hour. That is why, the at least one condition can be: a variable IOB(t) representative of the time variation of the user's quantity of insulin on board is lower than a predetermined value and/or a total amount of insulin calculated during a predetermined past time period is lower than a predetermined value.

If the at least one condition is not met, the calculation step is not implemented or the output of the calculation step is set to zero or is null. The output can also be: "no recommendation".

Periodicity

The compensation is implemented by the system after each predetermined period of time, for example each five minutes. In this embodiment, the estimated size of the unannounced meal corresponds to a deviation of blood glucose during five minutes. It is generally a small deviation which leads to a small estimated size and therefore a small bolus of insulin compared to a deviation for a longer period of time, for example 15 minutes, as it is generally the case.

That is why the estimation step and the calculation step are repeated by the estimation module 102 and the compensation module 103 for a determined number of times, for example four times in a row, with a determined time periodicity, for example five minutes.

The predetermined number of times can be comprised between 2 and 10, preferably between 3 and 5, even more preferably equal to 4 as described above.

The predetermined time periodicity is comprised between 1 and 15 minutes, preferably between 1 and 10 minutes, even more preferably equal to 5 minutes as described above.

This small period of time allows a fine management of the glucose as soon as an unannounced meal begins.

For example, an appetizer can last 20 minutes. Each five minutes, the compensation will be implemented and a corresponding estimated size of ingested meal will be determined and then used to manage the upcoming rise of glucose due to five minutes of the appetizer.

The system allows to manage the upcoming rise since the first period of time, here the first 5 minutes, therefore permitting a smooth management of the glucose by delivering insulin if needed based on the estimation of the unannounced meal ingested.

**Claims**

1. Method for estimating a size of an unannounced meal ingested by a user, the method being implemented by a system, said system comprising:

   - a storage module (301), said storage module storing a time series of glucose value of the user in a glucose storage,
   - an expectation module (302), said expectation module (302) determining an expected glucose variation for a given time point,
- an estimation module (11), said estimation module (11) estimating the size of the unannounced meal ingested based on:
- a variation difference between
- a measured glucose variation between a glucose value at the given time point and a glucose value at a time point of the time series anterior to the given time point,

the glucose values being acquired by a glycemia acquisition system or stored in the glucose storage; and

- the expected glucose variation between the glucose value at the given time point and the glucose value at the time point of the time series anterior to the given time point;
- at least one user parameter relative and personalized to the user.

2. Method for estimating a size of an unannounced meal ingested according to the preceding claim, where the expectation module (302) is determining the expected glucose variation at the given time point based at least on:

- an insulin sensitivity factor relative to the user, and/or
- an instantaneous consumption of insulin by the user.

3. Method for estimating a size of an unannounced meal ingested according to any of the preceding claims, wherein the user parameter relative and personalized to the user is based on the weight of the user.

4. Method for estimating a size of an unannounced meal ingested according to any of the preceding claims, wherein the system is further comprising a first estimation safety module (13a), said first estimation safety module (13a) determining:

- if the measured glucose variation determined by the estimation module (11) exceeds a variation difference threshold value and setting the measured glucose variation at a value equal to the variation difference threshold value if the measured glucose variation determined by the estimation module (11) exceeds the variation difference threshold value.

5. Method for estimating a size of an unannounced meal ingested according to any of the preceding claims 1 to 4, wherein the system is further comprising a second estimation safety module (13b), said second estimation safety module (13b) determining:

- if the size of the unannounced meal ingested estimated by the estimation module (11) exceeds a meal estimation threshold value and setting the size at a value equal to the meal estimation threshold value if the size of the unannounced meal ingested estimated by the estimation module (11) exceeds a meal estimation threshold value.

6. Method for estimating a size of an unannounced meal ingested according to the preceding claim wherein the meal estimation threshold value is calculated based on a threshold parameter determined based on the given time point.

7. Method for estimating a size of an unannounced meal ingested according to any of the preceding claims, wherein the system is further comprising a correction module (12),

said correction module (12) correcting the estimated size of the unannounced meal ingested estimated by the estimation module (11) according to at least one correction coefficient,
the at least one correction coefficient being predetermined according to a user profile based at least on an age, a weight, a sex of the user and/or insulin needs of the user.

8. Method for estimating a size of an unannounced meal ingested according to any of the preceding claims, wherein the system is further comprising an aggressiveness module (14),
said aggressiveness module (14) calculating a customized aggressiveness estimation of the size of the meal by applying an aggressiveness factor to the estimation size of the unannounced meal ingested estimated by the estimation module (11).

9. System for estimating a size of an unannounced meal ingested by a user comprising:

   - a storage module (301) adapted to store a time series of glucose value of the user in a glucose storage,
   - an expectation module (302) adapted to determine an expected glucose expected glucose variation for a given time point,
   - an estimation module (11) adapted to estimate the size of the unannounced meal ingested based on:
   - a variation difference between
   - a measured glucose variation between a glucose value at the given time point and a glucose value at a time point of the time series anterior to the given time point,

   the glucose values being acquired by a glycemia acquisition system or stored in the glucose storage; and

   - the expected glucose variation between the glucose value at the given time point and the glucose value at the time point of the time series anterior to the given time point;
   - at least one user parameter relative and personalized to the user.

10. System for estimating a size of an unannounced meal ingested by a user according to the preceding claim, wherein the system is further comprising a first estimation safety module (13a) adapted to:

   - determine if the variation difference determined by the estimation module (11) exceeds a variation difference threshold value and to set the variation difference at a value equal to the variation difference threshold value if the variation difference determined by the estimation module (11) exceeds the variation difference threshold value.

11. System for estimating a size of an unannounced meal ingested by a user according to any of the preceding claims 9 or 10, wherein the system is further comprising a second estimation safety module (13b) adapted to:

   - determine if the size of the unannounced meal ingested estimated by the estimation module (11) exceeds a meal estimation threshold value and to set the size at a value equal to the meal estimation threshold value if the size of the unannounced meal ingested estimated by the estimation module (11) exceeds a meal estimation threshold value. [12] System for estimating a size of an unannounced meal ingested by a user according to any of the preceding claim, wherein the meal estimation threshold value is calculated based on a threshold parameter determined based on the given time point.

12. System for estimating a size of an unannounced meal ingested by a user according to any of the preceding claims 9 to 12 further comprising a correction module (12) adapted to correct the estimated size of the unannounced meal ingested estimated by the estimation module (11) according to at least one correction coefficient, the at least one correction coefficient being predetermined according to a user profile based at least on an age, a weight, a sex of the user and/or insulin needs of the user.

13. System for estimating a size of an unannounced meal ingested by a user according to any of the preceding claims 9 to 13 further comprising an aggressiveness module (14) adapted to calculate a customized aggressiveness estimation of the size of the meal by applying an aggressiveness factor to the estimation size of the unannounced meal ingested estimated by the estimation module (11).

14. A computer program for estimating a size of an unannounced meal ingested by a user wherein the computer program is adapted, when run on a processor, to cause the processor to implement the method of the claim 1 to 8.

[Fig. 1]

BGI
module

$$BGI(t) = insulin\_activity(t) \times ISF(t) \times \Delta t$$

ISF
insulin_activity
$Gly(T_0)$
$Gly(T_0 - \Delta t)$

$$G(T_0) = Gly(T_0) - Gly(T_0 - \Delta t)$$
$$D(T_0) = G(T_0) - BGI(T_0)$$
$$CHO(T_0) = D(T_0) \times SugRatio(T_0) \times 100$$

estimation module 11

[Fig. 2]

$$CHOsafe(T_0) = min(CHOagr(T_0); CARBSMAX(T_0) - CHOtotal(T_0))$$

$\rightarrow$ CHOsafe$(T_0)$

estimation safety module 13

$$CHOagr(T_0) = CHOcorr(T_0) \times \frac{\beta}{\beta_0}$$

$\rightarrow$ CHOagr$(T_0)$

aggressiveness module 14

$$CHOcorr(T_0) = UMM\_A\_CORR \times CHO(T_0) + UMM\_B\_CORR$$

$\rightarrow$ CHOcorrr$(T_0)$

correction module 12

$\rightarrow$ CHO$(T_0)$

[Fig. 3]

21

5

4

3

2

1

[Fig. 4]

| | |
|---|---|
| 31 | |
| 32 | |
| | 302  301 |
| 11  12  13 (13a ;13b) | 14 |

3

[Fig. 5]

| |
|---|
| 101 |
| 103 |
| 102 |
| 32 |

1

Europäisches
Patentamt

European
Patent Office

Office européen
des brevets

# EUROPEAN SEARCH REPORT

Application Number

EP 23 19 6835

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FATHI ANAS EL ET AL: "An Unannounced Meal Detection Module for Artificial Pancreas Control Systems", 2019 AMERICAN CONTROL CONFERENCE (ACC), AMERICAN AUTOMATIC CONTROL COUNCIL, 10 July 2019 (2019-07-10), pages 4130-4135, XP033605328, * the whole document * | 1-14 | INV. A61B5/145 |
| X | US 2022/280720 A1 (EL FATHI ANAS [CA] ET AL) 8 September 2022 (2022-09-08) * the whole document * | 1-14 | |
| X | WO 2022/081788 A1 (UNIV VIRGINIA PATENT FOUNDATION [US]) 21 April 2022 (2022-04-21) * page 15, line 13 - page 39, line 2 * | 1,9,14 | |
| X | US 2022/273874 A1 (BRETON MARC D [US] ET AL) 1 September 2022 (2022-09-01) * paragraphs [0066] - [0081]; figures 7,8 * | 1,4,8,9, 14 | |
| X | SIKET MATE ET AL: "Automatically estimated meals in Model Predictive Control-Moving Horizon Estimation control strategy", 2022 13TH ASIAN CONTROL CONFERENCE (ASCC), ACA, 4 May 2022 (2022-05-04), pages 1367-1372, XP034152495, DOI: 10.23919/ASCC56756.2022.9828202 * the whole document * | 1,9,14 | TECHNICAL FIELDS SEARCHED (IPC) A61B |

-/--

The present search report has been drawn up for all claims

| Place of search | Date of completion of the search | Examiner |
|---|---|---|
| Berlin | 13 November 2023 | Kronberger, Raphael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 23 19 6835

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GODOY J L ET AL: "Meal detection and carbohydrate estimation based on a feedback scheme with application to the artificial pancreas", BIOMEDICAL SIGNAL PROCESSING AND CONTROL, ELSEVIER, AMSTERDAM, NL, vol. 68, 18 May 2021 (2021-05-18), XP086638853, ISSN: 1746-8094, DOI: 10.1016/J.BSPC.2021.102715 [retrieved on 2021-05-18] * the whole document * | 1,9,14 | |
| X | Eng Emilia Fushimi: "Artificial Pancreas: Evaluating the ARG Algorithm Without Meal Announcement", Journal of Diabetes Science and Technology Diabetes Technology Society, 22 March 2019 (2019-03-22), pages 1035-1043, XP055789905, Retrieved from the Internet: URL:https://journals.sagepub.com/doi/pdf/10.1177/1932296819864585 [retrieved on 2021-03-25] * the whole document * | 1,9,14 | |

TECHNICAL FIELDS
SEARCHED (IPC)

The present search report has been drawn up for all claims

| Place of search | Date of completion of the search | Examiner |
|---|---|---|
| Berlin | 13 November 2023 | Kronberger, Raphael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                     
& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 19 6835**

This annex lists the patent family members relating to the patent documents cited in the above-mentioned European search report.
The members are as contained in the European Patent Office EDP file on
The European Patent Office is in no way liable for these particulars which are merely given for the purpose of information.

**13-11-2023**

| Patent document cited in search report | | Publication date | Patent family member(s) | | Publication date |
|---|---|---|---|---|---|
| US 2022280720 | A1 | 08-09-2022 | AU | 2020310616 A1 | 20-01-2022 |
| | | | CA | 3146060 A1 | 14-01-2021 |
| | | | CN | 114127860 A | 01-03-2022 |
| | | | EP | 3997712 A1 | 18-05-2022 |
| | | | JP | 2022539818 A | 13-09-2022 |
| | | | US | 2022280720 A1 | 08-09-2022 |
| | | | WO | 2021005552 A1 | 14-01-2021 |
| WO 2022081788 | A1 | 21-04-2022 | AU | 2021360836 A1 | 15-06-2023 |
| | | | CA | 3195456 A1 | 21-04-2022 |
| | | | CN | 116670774 A | 29-08-2023 |
| | | | EP | 4228498 A1 | 23-08-2023 |
| | | | KR | 20230107245 A | 14-07-2023 |
| | | | WO | 2022081788 A1 | 21-04-2022 |
| US 2022273874 | A1 | 01-09-2022 | NONE | | |

For more details about this annex : see Official Journal of the European Patent Office, No. 12/82